# EUROPEAN PATENT SPECIFICATION

(11) **EP 2 016 970 B1**
(45) Date of publication and mention of the grant of the patent: **15.09.2010**
(21) Application number: 08160309.4
(22) Date of filing: 14.07.2008
(51) Int. Cl.: A61M 35/00, A61M 3/02

(54) **Device for washing wounds with dispenser spout suitable for tangential use.**
Vorrichtung zur Wundreinigung mit Spenderschnabel für tangentiale Verwendung
Dispositif pour le lavage de plaies avec bec verseur adapté pour une utilisation tangentielle

(30) Priority: 20.07.2007 IT MI20071459
(43) Date of publication of application: 21.01.2009
(73) Proprietor: GOBBI FRATTINI, Ditta Paolo Giuseppe, 23035 Sondalo (SO) (IT)
(72) Inventor: Apolet, Josek Berek, 20146, Milano (IT); Valoti, Michele, 6900, Massagno (CH)
(74) Representative: Mittler, Enrico

(56) References cited:
- US-A- 3 844 284
- US-A- 3 892 311
- US-A- 4 357 937
- US-A1- 2005 113 794
- US-A1- 2005 124 946

## Description

The present invention relates to a device for washing wounds with dispenser spout suitable for tangential use.

The problem of washing wounds, necroses and the like is not a commonplace one. An injured person who reaches a casualty ward before having been visited by a specialist doctor, is taken into charge by the on-duty nursing staff which, among its other duties, must clean the wound to make the injury visible to the specialist doctor. The current state of the art does not envisage a common guideline to perform this operation. The nursing staff therefore sees the cleaning of the wound left to its common sense, which varies from person to person. The paradoxical situation can be arrived at whereby, including due to excess zeal, the wound is cleaned with penetration of part of the dirt even further in depth, or also removing the layer of cells in mitotic phase, i.e., in regeneration, thus returning the healing process every time to stage one; if these operations are regularly repeated, as in the case of bed sores and chronic wounds, that have to be periodically cleaned, these risk never healing. For example, excessive pressure of the cleaning fluid or vigorous friction can produce the aforementioned effects on the regenerating cells.

In this respect, washing systems exist on the market that envisage the use of jets of water produced by more or less vigorous gas pressure, typically oxygen. Such jets cannot be produced at a sufficiently low pressure, and the presence of extraneous gases can be harmful for some tissues, oxygen for example being harmful for bone tissue; furthermore, the machinery is normally rather cumbersome and costly, not very suitable for and not always available in a casualty ward or in outdoor situations.

Bags exist on the market that can also be compressed by hand, containing cleaning liquids for various uses, including outdoor use, Nevertheless, the liquid dispenser spout is normally adapted to a specific use and no bag is available on the market that can also be compressed by hand, with portability characteristics and with a dispenser spout adapted for the present purpose. The ideal solution in fact is a flat and not cylindrical flow, that can be directed in a precise way, preferably in a tangential way to the surface to be cleaned, so that the dirt does not penetrate deeper with a water pressure perpendicular to the wound, but instead adds a tangential movement component that shifts the dirt and moves it to more suitable areas.

Also desirable is using the bag with just one hand, without interruption to close or change the pressure of the jet, which is regulated with just one hand which dispenses the liquid by pressing on the bag and at the same time directs the jet; this gives the nursing staff the chance to have one hand free for further emergency and cleaning operations.

The object of the present invention is to make a device for washing wounds and the like that can be handled with just one hand having characteristics of portability and liquid dispensing such as to be used in every situation, especially those requiring delicacy and precise direction of the jet of washing liquid.

According to the present invention, said object is achieved with a device for washing wounds and the like, comprising a flexible bag that can be pressure emptied containing a washing liquid, said bag comprising an inlet for filling the bag and an opening connected with a tube, and a dispenser spout suitable for tangential use connected to said tube and having an inlet portion and a tapered flat outlet portion, characterised in that said dispenser spout comprises in said tapered and flat portion at least one rectangular hole for the drainage of the washing liquid.

These and other characteristics of the present invention will be made more evident by the following detailed description of an example of practical embodiment illustrated in a non-limitative way on the attached drawings, of which;
the figure 1 shows an assembly view of the device according to the invention;
the figure 2 shows a section view according to the line II-II of the figure 1;
the figure 3 shows an axonometric view from below of the dispenser spout complete with closing cap;

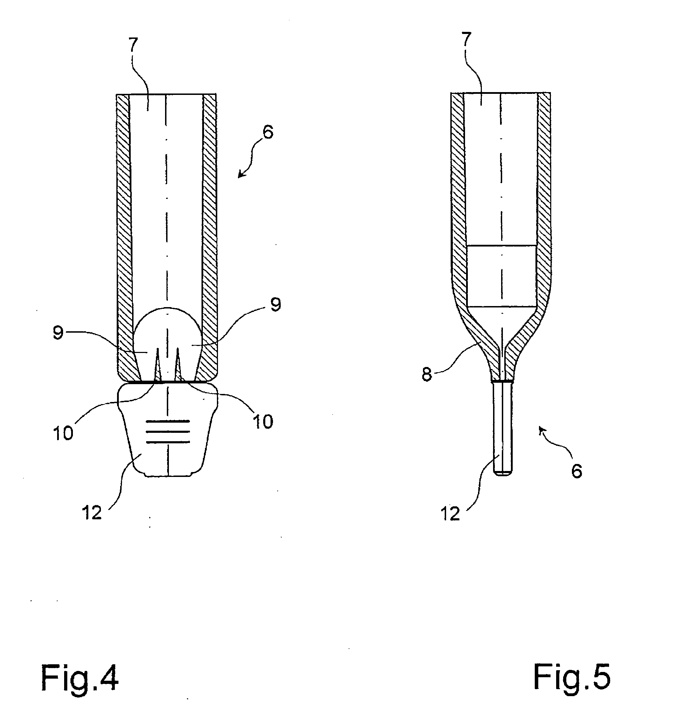

the figure 4 shows the dispenser cap in axial section, with cap;
the figure 5 shows the dispenser cap in axial section at 90° with respect to figure 3, with cap;
the figure 6 shows the dispenser spout sectioned as in the figure 4 but without cap;
the figure 7 shows a second view according to the line VII-VII of the figure 6;
the figure 8 shows a view of the figure 6 from below;
the figure 9 shows the dispenser spout in axial section at 90° with respect to figure 6;

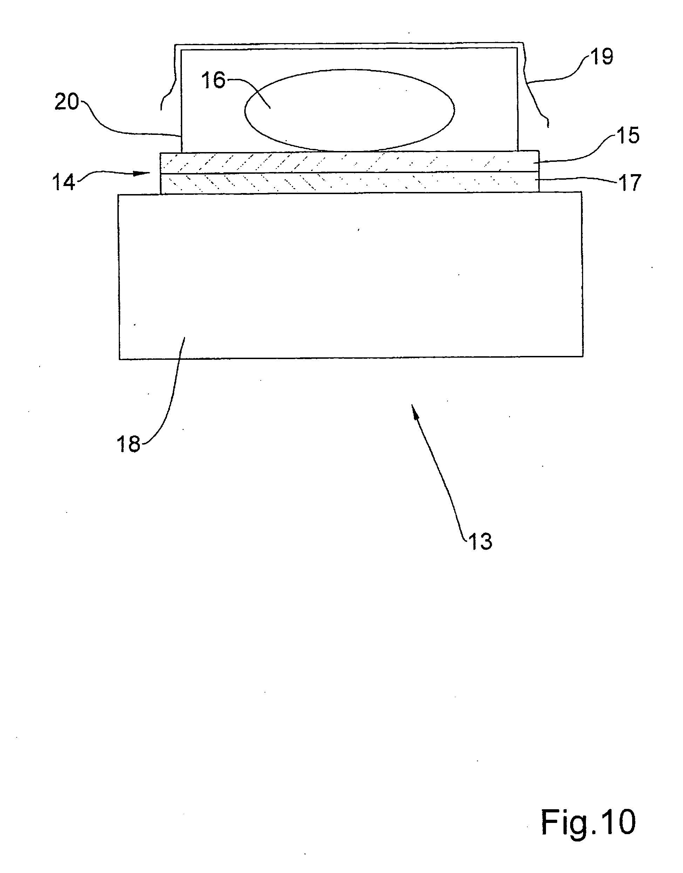

the figure 10 shows a schematic view of a kit for washing wounds,

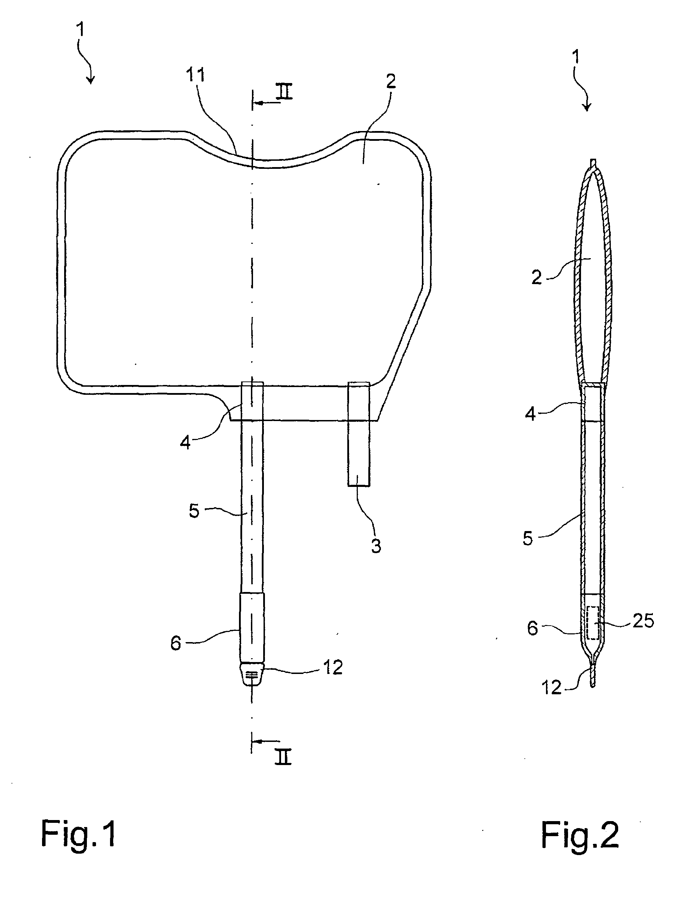

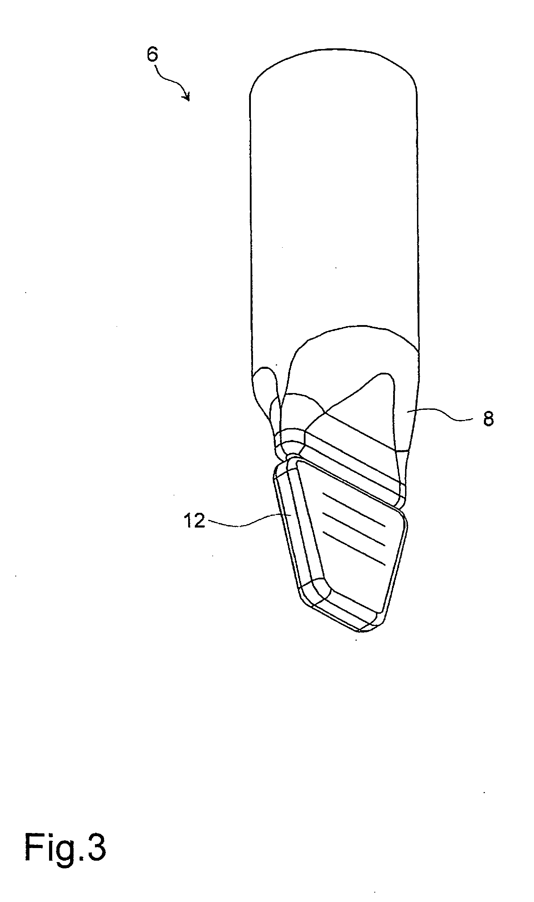

With reference to the figures 1 and 2 a flexible bag 1 will be noticed containing a washing liquid 2 for wounds, liquid obtainable according to the teachings available in the known state of the art, which is introduced through the inlet 3, which is sealed immediately after filling the bag 2, to maintain its sterility characteristics. Through an opening 4, a tube 5, which advantageously can be flexible, connects the bag to a tangential dispenser spout 6, advantageously having a cap 12 frangible at the time of use (fig. 3).

Now with reference to the figures from 4 to 9, it will be seen that the dispenser spout 6 comprises an inlet portion 7 for the introduction of the washing liquid 2 into the dispenser spout 6 and the connection to the tube 5, and a tapered flat portion 8 that favours the conformation of the exiting flow of washing liquid 2 according to a substantially flat jet, which can be directed tangentially to the wound with the aforementioned advantages.

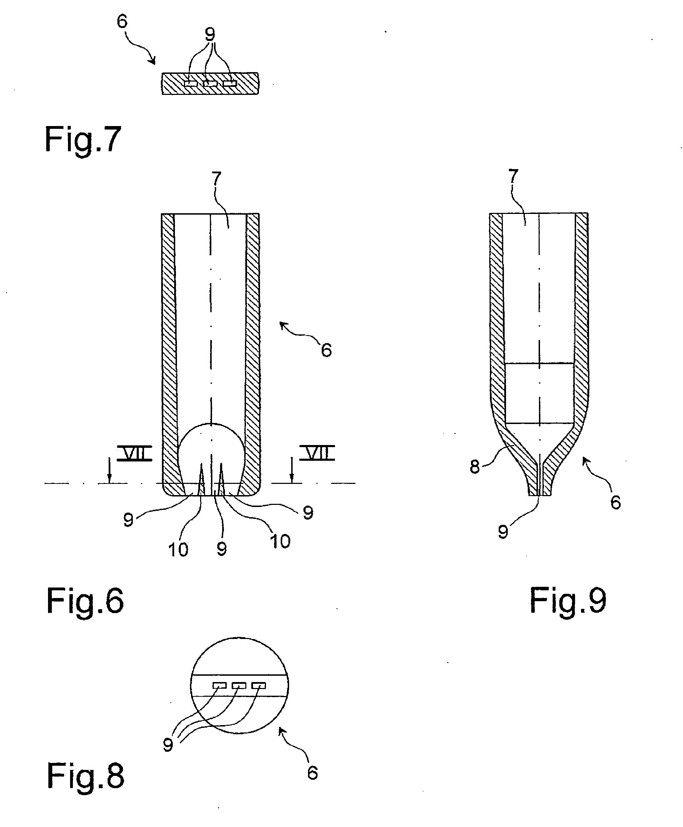

In particular in the figures from 6 to 8 an embodiment will be noticed of the dispenser spout 6 that comprises three rectangular holes 9, having rectangular section, with axes substantially parallel to the axis of the dispenser spout 6, and arranged in a straight line. With particular reference to the figure 6, it will be seen that the rectangular holes 9 are separated for a section, inside the dispenser spout 6, by invitation plates 10, for the purpose, together with the shape of the holes, of breaking into minor modules any turbulence of the exiting liquid and maximising the movement component in the direction of the axis of the dispenser spout. To the greater advantage of the above objects, the invitation plates 10 can be wedge shaped, as shown for example in the figure 6.

It must be emphasised that the cap 12 is easily frangible by hand at the time of use by turning it around the axis of the dispenser spout 6., Advantageously, in place of the outer cap 12 a hermetic, sealing element 25 can be present in the spout 6 that is frangible with the bending of the spout 6 at the time of use, of the type, for example, already used for the patent IT 1339433, of the same holder, and which is schematised by the broken line in the figure 2.

Advantageously the bag 1 can be emptied by hand by squeezing it and the shape of the bag 1 can include a recess 11 for a stronger grip (fig. 1). The bag 1 can also be emptied by applying the pressure of an external gas.

Advantageously, the device for washing wounds can include a downflow device (not shown, and for example of the type already used for the patent IT 1339433 of the same holder) on the tube 5, between the bag 1 and the spout 6, suitable for regulating the downflow speed of the washing liquid.

Advantageously, the device for washing wounds can include a needle point (not shown) placed at the inlet 3, suitable for introducing drugs, including but not limited to, antibiotics and/or cicatrizants, by means of a syringe.

Advantageously, the filling of the bag 1 can also be done through the opening 4.

Bag 1 and tube 5 are made of flexible material and the spout 6 of soft material, e.g., PVC has these characteristics.

Advantageously, the device for washing wounds can form part of a kit 13 for washing wounds that comprises a first double-layer fabric 14 to be placed underneath an injured person 16 with a first absorbing layer 15 to be turned towards the injured person 16 and a second waterproof layer 17 in contact with a couch 18, a second fabric 19 in completely transparent waterproof material or having a transparent window, not shown, a frame 20 with eight elements that can be assembled in an articulated way with quick coupling for supporting the second fabric 19 above the wound leaving free strips of fabric, so as to allow access to a first-aid worker who can clean the wound without coming into contact with the consequent sprays.

Further elements that can be advantageously included in the kit 13, and which are not shown, are: sterile protection gloves, plastic forceps with sterile rings, portions of sterile gauze to dab the wound.

The whole is completed by a bag, not shown, to dispose of the used kit in accordance with the law.

## Claims

1. Device for washing wounds and the like, comprising a flexible bag (1), that can be emptied by pressure, containing a washing liquid (2), said bag (1) comprising an inlet (3) for filling the bag (1) and an opening (4) connected to a tube (5), and a dispenser spout (6) suitable for tangential use on the wound connected to said tube (5) and having an inlet portion (7) and a tapered flat outlet portion (8), **characterised in that** said dispenser spout (6) comprises in said flat and tapered portion (8) at least one rectangular hole (9) for draining the washing liquid (2).

2. Device according to claim 1, **characterised in that** said dispenser spout (6) comprises a plurality of rectangular holes (9) with bigger axes substantially parallel with respect to the bigger axis of the dispenser spout (6) and arranged in a straight line.

3. Device according to claim 2, **characterised in that** the rectangular holes (9) are separated from one another by invitation plates (10) inside the spout (6) to improve the alignment of the flow with the spout axis (6).

4. Device according to claim 3, **characterised in that** the invitation plates (10) are wedge shaped.

5. Device according to any one of the previous claims, **characterised in that** said bag (1) has an anatomic shape with a recess (11) to be able to firmly grip the bag (1) by hand and empty it with just one hand.

6. Device according to any one of the previous claims, **characterised in that** said bag (1) allows emptying with the application of a pressure of an external gas.

7. Device according to any one of the previous claims, **characterised in that** the tube (5) is flexible.

8. Device according to any one of the previous claims, **characterised in that** the spout (6) is made of soft material.

9. Device according to claim 8, **characterised in that**, internally, the spout (6) has a hermetic, sealing element (25) frangible with the bending of the spout (6) at the time of use to maintain the sterility of the washing liquid (2).

10. Device according to any of the claims from 1 to 8 **characterised in that** the spout (6) has a closing cap (12) frangible by hand at the time of use to maintain the sterility of the washing liquid (2).

11. Device according to any one of the previous claims, **characterised in that** a downflow device is fitted on the tube (5), between the bag (1) and the spout (6), suitable for regulating the downflow speed of the washing liquid.

12. Device according to any one of the previous claims, **characterised in that** a needle point is fitted on the inlet (3), suitable for the introduction of drugs by means of a syringe.

13. Device according to any one of the previous claims, **characterised in that** the filling of the bag (1) can be effected through the opening (4).

14. Device according to claim 13, **characterised in that** the opening (4) is sealed after opening the bag (1).

15. Device according to any of the claims from 2 to 14, **characterised in that** there are three rectangular holes (9).

16. Device according to any one of the previous claims, **characterised in that** it is made of PVC.

17. Kit (13) for washing wounds comprising the washing device according to any one of the previous claims and a device for covering and protecting the washing area suitable for allowing the access of a first-aid worker who can clean the wound without coming into contact with the consequent sprays.

18. Kit (13) according to claim 17, **characterised in that** it includes a first double-layer fabric (14) to be placed underneath an injured person (16) with a first absorbent layer (15) to be turned towards the injured person (16) and a second waterproof layer (17) in contact with a couch (18), a second fabric (19) in waterproof material, a frame (20) for supporting the second fabric (19) above the wound leaving some strips of fabric free for the first-aid worker to be able to access the injured person (16).

19. Kit (13) according to claim 18, **characterised in that** the second fabric (19) has a transparent window.

20. Kit (13) according to claim 18, **characterised in that** the second fabric (19) is completely transparent.

21. Kit (13) according to any of the claims from 18 to 20, **characterised in that** the frame (20) has eight elements that can be assembled in an articulated way with quick coupling.

22. Kit (13) according to any of the claims from 17 to 21, **characterised in that** it also includes sterile protection gloves, plastic forceps with sterile rings, portions of sterile gauze and a bag for disposing of the remaining components of the kit (13) after use.

## Patentansprüche

1. Vorrichtung zum Waschen von Wunden und dergleichen, mit einem flexiblen Beutel (1), der durch Druck entleert werden kann und eine Waschflüssigkeit (2) enthält, wobei der Beutel (1) einen Einlass (3) zum Füllen des Beutels (1) und eine Öffnung (4) aufweist, die mit einer Röhre (5) verbunden ist, und einer Abgabetülle (6), die für eine tangentiale Verwendung an der Wunde verwendbar ist, mit der Röhre (5) verbunden ist und einen Einlassabschnitt (7) sowie einen abgeschrägten, flachen Auslassabschnitt (8) hat, **dadurch gekennzeichnet, dass** die Abgabetülle (6) in dem flachen und abgeschrägten Abschnitt (8) zumindest ein rechteckiges Loch (9) zum Auslassen der Waschflüssigkeit (2) aufweist.

2. Vorrichtung gemäß Anspruch 1, **dadurch gekennzeichnet, dass** die Abgabetülle (6) eine Vielzahl an rechteckigen Löchern (9) mit größeren Achsen aufweist, die im Wesentlichen parallel bezüglich der größeren Achse der Abgabetülle (6) sind, und die entlang einer Geraden angeordnet sind.

3. Vorrichtung gemäß Anspruch 2, **dadurch gekennzeichnet, dass** die rechteckigen Löcher (9) durch Einladungsplatten (10) im Inneren der Tülle (6) voneinander getrennt sind, um die Ausrichtung der Strömung an die Tüllenachse (6) zu verbessern.

4. Vorrichtung gemäß Anspruch 3, **dadurch gekennzeichnet, dass** die Einladungsplatten (10) keilförmig sind.

5. Vorrichtung gemäß einem der vorherigen Ansprüche, **dadurch gekennzeichnet, dass** der Beutel (1) eine anatomische Form mit einer Ausnehmung (11) hat, um den Beutel (1) mit der Hand fest greifen zu können und ihn mit nur einer Hand zu entleeren.

6. Vorrichtung gemäß einem der vorherigen Ansprüche, **dadurch gekennzeichnet, dass** der Beutel (1) eine Entleerung durch Aufbringen eines Drucks von einem externen Gas erlaubt.

7. Vorrichtung gemäß einem der vorherigen Ansprüche, **dadurch gekennzeichnet, dass** die Röhre (5) flexibel ist.

8. Vorrichtung gemäß einem der vorherigen Ansprüche, **dadurch gekennzeichnet, dass** die Tülle (6) aus einem weichen Material besteht.

9. Vorrichtung gemäß Anspruch 8, **dadurch gekennzeichnet, dass** die Tülle (6) im Inneren ein hermetisches Dichtelement (25) hat, das beim Biegen der Tülle (6) während des Gebrauchs zerbrechlich ist, um die Sterilität der Waschflüssigkeit (2) aufrechtzuerhalten.

10. Vorrichtung gemäß einem der Ansprüche 1 bis 8, **dadurch gekennzeichnet, dass** die Tülle (6) eine Verschlusskappe (12) hat, die durch die Hand während des Gebrauchs zerbrechlich ist, um die Sterilität der Waschflüssigkeit (2) aufrechtzuerhalten.

11. Vorrichtung gemäß einem der vorherigen Ansprüche, **dadurch gekennzeichnet, dass** eine Abströmvorrichtung an der Röhre (5) zwischen dem Beutel (1) und der Tülle (6) angebracht ist, die zum Regulieren der Abströmgeschwindigkeit der Waschflüssigkeit geeignet ist.

12. Vorrichtung gemäß einem der vorherigen Ansprüche, **dadurch gekennzeichnet, dass** ein Nadelpunkt an dem Einlass (3) angebracht ist, der für die Einführung von Arzneimitteln mittels einer Spritze geeignet ist.

13. Vorrichtung gemäß einem der vorherigen Ansprüche, **dadurch gekennzeichnet, dass** das Füllen des Beutels (1) durch die Öffnung (4) bewirkt werden kann.

14. Vorrichtung gemäß Anspruch 13, **dadurch gekennzeichnet, dass** die Öffnung (4) nach dem Öffnen des Beutels (1) abgedichtet ist.

15. Vorrichtung gemäß einem der Ansprüche 2 bis 14, **dadurch gekennzeichnet, dass** drei rechteckige Löcher (9) vorhanden sind.

16. Vorrichtung gemäß einem der vorherigen Ansprüche, **dadurch gekennzeichnet, dass** sie aus PVC besteht.

17. Kit (13) zum Waschen von Wunden, das die Waschvorrichtung gemäß einem der vorherigen Ansprüche und eine Vorrichtung zum Abdecken und Schützen des Waschbereichs aufweist, der zum Ermöglichen eines Zugangs eines Ersthelfers geeignet ist, der die Wunde reinigen kann, ohne dass er mit den nachfolgenden Sprays in Kontakt gelangt.

18. Kit (13) gemäß Anspruch 17, **dadurch gekennzeichnet, dass** es ein erstes doppellagiges Gewebe (14), das unter einer verletzten Person (16) zu platzieren ist, wobei eine erste Absorptionslage (15) zu der verletzten Person (16) gewandt ist und eine zweite wasserdichte Lage (17) mit einer Liege (18) in Kontakt ist, ein zweites Gewebe (19) aus einem wasserdichten Material, und einen Rahmen (20) zum Stützen des zweiten Gewebes (19) über der Wunde aufweist, der einige Gewebestreifen für den Ersthelfer freilässt, damit er einen Zugang zu der verletzten Person (16) haben kann.

19. Kit (13) gemäß Anspruch 18, **dadurch gekennzeichnet, dass** das zweite Gewebe (19) ein transparentes Fenster hat.

20. Kit (13) gemäß Anspruch 18, **dadurch gekennzeichnet, dass** das zweite Gewebe (19) vollständig transparent ist.

21. Kit (13) gemäß einem der Ansprüche 18 bis 20, **dadurch gekennzeichnet, dass** der Rahmen (20) acht Elemente hat, die mit Schnellkupplungen gelenkig zusammengebaut werden können.

22. Kit (13) gemäß einem der Ansprüche 17 bis 21, **dadurch gekennzeichnet, dass** es außerdem sterile Schutzhandschuhe, Plastikpinzetten mit sterilen Ringen, Abschnitte aus sterilem Mull und einen Beutel zum Ablegen der übrigen Komponenten des Kits (13) nach dem Gebrauch beinhaltet.

## Revendications

1. Dispositif pour le lavage de plaies et similaires, comprenant une poche flexible (1), qui peut être vidée par pression, qui contient un liquide de lavage (2), ladite poche (1) comprenant une entrée (3) pour remplir la poche (1) et une ouverture (4) reliée à un tube (5), et un bec distributeur (6) qui convient pour l'utilisation tangentielle sur la blessure, connecté sur ledit tube (5) et ayant une portion d'entrée (7) et une portion de sortie (8) plane effilée, **caractérisé en ce que** le bec distributeur (6) comprend au moins un trou rectangulaire (9) dans ladite portion plane effilée (8) pour drainer le liquide de lavage (2).

2. Dispositif selon la revendication 1, **caractérisé en ce que** ledit bec distributeur (6) comprend une pluralité de trous rectangulaires (9) avec des grands axes sensiblement parallèles par rapport au grand axe du bec (6) du distributeur et agencés sur une ligne droite.

3. Dispositif selon la revendication 2, **caractérisé en ce que** les trous rectangulaires (9) sont séparés les uns des autres par des plaques d'invitation (10) à l'intérieur du bec (6) pour améliorer l'alignement et l'écoulement sur l'axe (6) du bec.

4. Dispositif selon la revendication 3, **caractérisé en ce que** les plaques d'invitation (10) sont en forme de coin.

5. Dispositif selon l'une quelconque des revendications précédentes, **caractérisé en ce que** ladite poche (1) a une forme anatomique avec un évidement (11) pour être capable de saisir fermement la poche (1) à la main et de la vider avec une seule main.

6. Dispositif selon l'une quelconque des revendications précédentes, **caractérisé en ce que** ladite poche (1) permet le vidage avec l'application d'une pression d'un gaz extérieur.

7. Dispositif selon l'une quelconque des revendications précédentes, **caractérisé en ce que** le tube (5) est flexible.

8. Dispositif selon l'une quelconque des revendications précédentes, **caractérisé en ce que** le bec (6) est réalisé en matériau souple.

9. Dispositif selon la revendication 8, **caractérisé en ce que**, à l'intérieur, le bec (6) possède un élément d'étanchement hermétique (25) capable d'être brisé par cintrage du bec (6) au moment de l'utilisation pour maintenir la stérilité du liquide de lavage (2)

10. Dispositif selon l'une quelconque des revendications 1 à 8, **caractérisé en ce que** le bac (6) possède un capuchon de fermeture (12) capable d'être brisé à la main au moment de l'utilisation pour maintenir la stérilité du liquide de lavage (2).

11. Dispositif selon l'une quelconque des revendications précédentes, **caractérisé en ce qu'**un dispositif de contrôle d'écoulement est monté sur le tube (5), entre la poche (1) et le bec (6), approprié pour réguler la vitesse d'écoulement du liquide de lavage.

12. Dispositif selon l'une quelconque des revendications précédentes, **caractérisé en ce qu'**une pointe à aiguille est montée sur l'entrée (3), appropriée pour l'introduction de médicaments au moyen d'une seringue.

13. Dispositif selon l'une quelconque des revendications précédentes, **caractérisé en ce que** le remplissage de la poche (1) peut être effectué à travers l'ouverture (4).

14. Dispositif selon la revendication 13, **caractérisé en ce que** l'ouverture (4) est scellée après ouverture de la poche (1).

15. Dispositif selon l'une des revendications 2 à 14, **caractérisé en ce qu'**il est prévu trois trous rectangulaires (9).

16. Dispositif selon l'une quelconque des revendications précédentes, **caractérisé en ce qu'**il est réalisé en PVC.

17. Trousse (13) pour le lavage de blessures, comprenant le dispositif de lavage selon l'une quelconque des revendications précédentes et un dispositif pour couvrir et protéger la zone de lavage, approprié pour permettre l'accès d'une personne chargée des premiers secours qui est en mesure de nettoyer la blessure sans venir en contact avec les produits pulvérisés concernés.

18. Trousse selon la revendication 17, **caractérisée en ce qu'**elle inclut un premier tissu à double couche (14) à placer au-dessous d'une personne blessée (16) avec une première couche absorbante (15) à tourner en direction de la personne blessée (16) et une seconde couche étanche à l'eau (17) en contact avec un matelas (18), un second tissu (19) en matériau étanche à l'eau, un cadre (20) pour supporter le second tissu (19) au-dessus de la blessure en laissant quelques rubans de tissus libres pour que la personne chargée des premiers secours soit capable d'accéder à la personne blessée (16).

19. Trousse (13) selon la revendication 18, **caractérisée en ce que** le second tissu (19) présente une fenêtre transparente.

20. Trousse (13) selon la revendication 18, **caractérisée en ce que** le second tissu (19) est complètement transparent.

21. Trousse (13) selon l'une quelconque des revendications 18 à 20, **caractérisée en ce que** le cadre (20) possède huit éléments qui peuvent être assemblés d'une manière articulée avec accouplement rapide.

22. Trousse (13) selon l'une quelconque des revendications 17 à 21, **caractérisée en ce qu'**elle inclut également des gants de protection stériles, des forceps en matière plastique avec bagues stériles, des portions de gaze stériles et une poche pour jeter les composants restants de la trousse (13) après utilisation.
